# EUROPEAN PATENT SPECIFICATION

(11) **EP 1 433 481 B1**
(45) Date of publication and mention of the grant of the patent: **17.05.2006**
(21) Application number: 03405852.9
(22) Date of filing: 01.12.2003
(51) Int. Cl.: A61K 31/568, A61P 31/20, A61P 17/12

(54) **The use of methyltestosterone for the treatment of human papilloma virus infections**
Verwendung von Methyltestosteron zur Behandlung von menschlichen Papillomavirus Infektionen
Utilisation de methyltestosterone pour le traitement d'une infection par le virus du papillome humain

(30) Priority: 23.12.2002 CN 02128078
(43) Date of publication of application: 30.06.2004
(73) Proprietor: Liang, Youguo, Chengdu City, Sichuan Province, 610015 (CN)
(72) Inventor: Youguo, Liang, Chengdu City Sichuan Province, 610015 (CN); Xiaohong, Xie, Chengdu City Sichuan Province, 610015 (CN); Zhiyu, Xie, Chengdu City Sichuan Province, 610015 (CN)
(74) Representative: Liebetanz, Michael

(56) References cited:
- DE-A- 3 514 724
- US-A- 4 605 648
- DATABASE WPI Section Ch, Week 200362 Derwent Publications Ltd., London, GB; Class B04, AN 2003-646980 XP002270745 & CN 1 424 040 A (LIANG Y), 18 June 2003 (2003-06-18)

## Description

### TECHNICAL FIELD

The present invention relates to the use of methyltestosterone for the manufacture of a drug for treatment of human papilloma virus (HPV) infections, and especially for treatment of verruca planae and condyloma acuminatum and the relapse of these diseases.

### BACKGROUND ART

The HPV virus is a non-enveloped, double-stranded, circular DNA virus made of 7900 base pairs from the family Papovaviridae, genus papillomavirus. It has an icosahedral capsid made of 72 capsometers and is 50-55 nm in diameter.

It is known that HPV cannot be cultured in cells nor multiply in animal models and that humans are the only host for HPV. As a result, little was known about the virology of HPV until the development of molecular biology in early 80's. Since then a great deal of research has been carried out on the disease mechanism caused by HPV.

Wart is a dermatosis that manifests as vegetations on the skin or mucous membrane. It has been postulated that HPV is the pathogen for warts as early as 1907. The hypothesis was later confirmed by the fact that healthy volunteers could be infected by the extract of wart tissues. It is now known that verruca planae and condyloma acuminatum are caused by HPV infection. There are 77 subtypes of HPV that may relate to wart-like dermatosis. Subtypes 1, 2, 3, 4, 10, 11, 18 and 28 selectively infect the skin or mucous membrane of humans and manifest as benign vegetations. HPV are transmitted primarily by direct contacts and can also be transmitted by contacting contaminated materials. Immuno-deficient and trauma patients are especially susceptible to HPV infections. The clinical manifestations are four types: verruca vulgaris, verruca planae, verruca plantaris and condyloma acuminatum. Verruca planae is a non-sexual transmitted disease (STD) with high incident rate. Condyloma acuminatum is a STD with a growing rate of occurance. In China, Approximately 25% of STD patients in China are caused by condyloma acuminatum. Condyloma acuminatum is also a common cause of STD in Europe and the USA, accounting for the third highest causes of all STD cases, only behind the cause of gonorrhea and mycoplasm, according to a report from the United Kingdom. According to the World Health Organization (WHO), there are about 30 million new cases of condyloma acuminatum each year and sub-clinical cases are even more prevalent.

Because the human is the only host for HPV, it is very difficult to develop drugs to treat verruca planae and condyloma acuminatum. DE 35 14 724A disclosed creams containing testosterone for the treatment of verruca or warts. Four pharmaceutical ingredients are contained in such creams, ie. vitamins, such as vitamine A, B6, C, D2, E, K3; progesterone; testosterone propionate; mentholi and sulfuri precipatati. However, the functions of these ingredients, the type of verrucas to be treated, and the mechanisms underlined are not disclosed. A large of our tests showed there is no effective case when using creams containing testosterone to treat verruca planae and condyloma acuminatum.

The current treatment uses immuno-modulators and non-specific anti-viral medications. These treatments have many disadvantages, among which are a long-term usage, uncertain results, high costs, and inconvenience in using.

The pathogenesis of HPV is complicated. HPV is not only the pathogen of warts but also may underlie the development of cervical cancer, carcinoma of penis, esophageal cancer, skin carcinoma and other squamous carcinomas. Because the human is the only host of HPV and it is not feasible to culture HPV in cells or animal models, efficacious and specific drugs of anti-HPV are slow to develop.

Professor Zhiyu Xie, one of the inventors, hypothesized that verruca planae caused by HPV might relate to the level of gonadal hormones after studies of a large number of clinical cases. Using methyltestosterone as a treament, she provides evidence that estrogen is an important causal factor of verruca planae. The other related papers and books are:
Cui M. H. Study on HPV and the female genitals' condyloma acuminata and its sub-clinical infection. Natl. Med. J. China, 1993, 7(9): 560-561;
Zhen H. et al. Measuring T cell's subtypes and the soluble interleukin II receptors of the peripheral blood of condyloma acuminata, Chin. J. Derm., 1993, 22:179-181;
Liu Z.F. et al. Measuring T cell and its subtypes of the peripheral blood of condyloma acuminata, Chin. J. Derm., 1990, 23: 339;
Dong Yi, Zhen Xuejun, Endocrinology & Metabology of Peking Unio Medical College, 1999, 1758-1760;
Deng Jieying, Endocrinology & Metabology of Peking Unio Medical College 1999, 144-145;
Wu Housheng, Qing Huiliang, Medical Immunology, 2000.10; 96.

Later on, using polymerase chain reaction (PCR) technology to study condyloma acuminatum caused by HPV suggests that estrogen receptor (ER) positive granules are mainly distributed in the granular layer, stratum spinosum and koilocytes.(Cui MH. Study on HPV and the female genitals' condyloma acuminata and its sub-clinical infection. (Natl. Med. J. China, 1993; 7(9): 560-561) The distribution of ER positive granules coincides with the distribution of koilocytes analyzed by histology, viral granules observed under electron microscope, and viral antigen protein analyzed by in situ hybridization. A high level of estrogen receptors is apparently correlated to a high rate of infection by HPV. These studies substantiate Professor Xie's hypothesis in which HPV infection relates to the level of estrogen and support a use of methyltestosterone for treatment of verruca planae.

Methyltestosterone is a derivative of testosterone with the C₁₇ of the chemical structure substituted by a methyl group. It is a classical drug that has functions of androgen and protein synthesis and has been in use for several decades. The chemical structure is as follows:

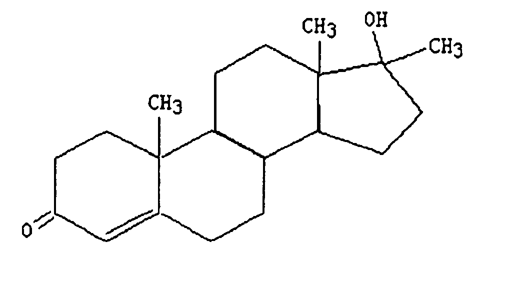

There is no disclosure till now that Methyltestosterone can be used as a pharmaceutical for treatment of HPV infections.

### DISCLOSURE OF THE INVENTION

According to the hypothesis, the present invention utilizes methyltestosterone for the manufacture of a drug to treat diseases infected by HPV, exemplified by verruca planae and condyloma acuminatum. The mechanism of action is to reduce the action of estrogen, thus inhibiting HPV multiply in cells of granular layer and stratum spinosum. Meanwhile, methyltestosterone can stimulate thymus proliferation, thymosin secretion, T cell maturation in thymus, and increase CD8 (+) T lymphocytes (CTL). CTLs can get into the cells of granular layer and stratum spinosum through the pores formed by perfurin, lyses cells infected by HPV, and thus kill HPV. This effect does not destroy normal cells in granular layer and stratum spinosum. Therefore, there are no necrosis and exfoliation of the wart and the cutin.

The present invention provides a use of androgenic hormone for the manufacture of a pharmaceutical for treatment of HPV infections, and provides evidence for effective and reliable treatment of verruca planae, condyloma acuminatum, and especially for treatment of relapses of condyloma acuminatum after systematic, physical, chemical, and operational treatments.

The present invention also provides a use of methyltestosterone for the manufacture of a pharmaceutical for treatment of HPV infections, and provides evidence for effective and reliable treatment of verruca planae, condyloma acuminatum, and especially for treatment of relapses of condyloma acuminatum after systematic, physical, chemical, and operational treatments.

It is a further objective of the invention to provide a use of methyltestosterone for the manufacture of a pharmaceutical for treatment of verruca planae.

It is a further objective of the invention to provide a use of methyltestosterone for the manufacture of a pharmaceutical for treatment of condyloma acuminatum.

In the present invention, said pharmaceutical with methyltestosterone can be administered orally or hypoglossally.

In the present invention, said methyltestosterone is comprised in a tablet preferably.

In the present invention, the content of methyltestosterone in said tablet is 5 mg/tablet.

Preferably, said tablet is administered hypoglossally, 1 tablet/time, two or three times/day, for 3-5 days consecutively.

Preferably, said tablet is administered orally, 1 tablet/time, three times/day, and 5 days consecutively, followed by 1 tablet/time, two times/day, for additional 5-10 days consecutively.

### DETAILED DESCRIPTION OF THE EMBODIMENTS

While particular embodiments of the invention have been particularly described hereinabove, it will be appreciated that the present invention is not limited thereto, since as will be readily apparent to skilled persons, many variations and modifications can be made. Such variations and modifications which have not been detailed herein are deemed to be obvious equivalents of the present invention. For example, structural analogs of methyltestosterone which substantially imitate the function of methyltestosterone in the human body are deemed to be obvious chemical equivalents of methyltestosterone. The essential concept, spirit and scope of the present invention will be better understood in the light of the claims which follow.

### Example 1

The study protocol using methyltestosterone as a treatment for verruca planae and condyloma acuminatum caused by HPV includes:

### Criteria of patient selection

### (1) Verruca planae.

The patients had characteristics of skin lesions of verruca planae. The exanthems scattered on the face, neck, and hands with 20 or more lesion spots, and were not treated by laser and freezing methods. The disease course was between 3 months to 10 years. The patients were treated with 5Fu ointment or 5% formalin topically, anti-viral drugs orally, or intra-muscular injection of herbal drugs, or with transferor, interferon, interleukin II. None of the patients were responsive to the therapy.

### (2) Condyloma acuminatum.

The patients displayed vegetations of typical condyloma acuminatum, positive for the acetowhitening test or for HPV antibodies, or treated by CO₂ laser and freezing methods. Some of the patients were the relapsed ones after the treatment by transferor, interferon, and interleukins.

### Drug regimen

Methyltestosterone is a tablet, manufactured by Southwest Pharmaceutical Company and the Second Pharmaceutical Company of Chengdu, China. The dosage was 5mg/tablet.

### Treatment protocol

### (1) Verruca planae.

Methyltestosterone was administered hypoglossally, 1 tablet/time, two or three times/day, for 3-5 days consecutively. The exanthems would all regress within five weeks after the above therapeutic regimen. If the exanthems did not regress completely, the treatment was repeated once more.

### (2) Condyloma acuminatum.

Methyltestosterone was administered orally, 1 tablet/time, three times/day, and 5 days consecutively, followed by 1 tablet/time, two times/day, for additional 5-10 days consecutively.

### Criteria of evaluating the therapeutic effect

### (1) Verruca planae.

All exanthems on the face regressed would be considered as cure, and 70% of them disappeared would be considered as effective. 70% of Exanthems on the hand and neck regressed would be considered as cure.

### (2) Condyloma acuminatum.

One of the following would be considered as cure: A) there were no new vegetations at or around the lesion for at least six months after administration of methyltestosterone for 10-15 days following treatment with physical, chemical, and operational therapies. B) the vegetations reappeared in two months after operations and were positive for the acetowhitening test, but regressed for at least 6 months after administration of methyltestosterone for 10-15 days.

### Results

### (1) Verruca planae.

103 patients, 45 males and 58 females, were treated. 96 patients with facial verruca planae and 7 with hand and neck verruca planae. They were between ages 16-56 years old. 85 of 96 cases with facial verruca planae were cured and 9 were effective, while 2 were non-responsive to the medication. The cure rate was 88.5%, effective rate was 9.4%, and non-responsive rate was 2%. 1 out of 7 cases with hand and neck verruca planae was cured, 3 regressed after 5 weeks following the treatment, and 1 remained non-responsive. The effective rate was 86%. So the treatment for the hand and neck verruca planae was less effective than that of the facial verruca planae.

### (2) Condyloma acuminatum.

61 patients with condyloma acuminatum were treated, including 46 males and 15 females. They were between ages 24-49 years old and administered methyltestosterone for 10-15 days following operations. None of the patients relapsed after 6 months to 1 year of the treatment. The two relapsed cases following operation and cytokine treatments were cured upon methyltestosterone treatment.

The relapse rate of condyloma acuminatum that was not given immuno-modulators after operations is 30-40%. However, there is no relapse case treated with methyltestosterone during the course of the study.

### Example 2

Typical cases of verruca planae and Condyloma acuminatum treated with methyltestosterone are shown as follows:
Case 1: Male, 21 years old with facial verruca planae for 2 years. He was given orally or intra-muscularly with indigowood roots or intra-muscularly with Vitamin B12, once per week for 8 weeks. He also took methenamine orally, herbal medications, and used formalin and 5Fu ointment topically. But all treatments had failed. After given methyltestosterone 1 tablet twice daily for 3 days, the patient was cured in 2 weeks.
Case 2: Female, 19 years old with facial verruca planae for 1 year. She was non-responsive to treatments of anti-viral drugs and indigowood roots. After given methyltestosterone 1 tablet twice daily for 3 days. All lessions regressed in 4 weeks.
Case 3: Male, 35 years old with facial verruca planae for 4 years but was non-responsive to a variety of treatments. After given methyltestosterone 1 tablet twice daily for 3 days, all lesions regressed in 4 weeks.
Case 4: Female, 42 years old with facial verruca planae for 13 years. After given methyltestosterone 1 tablet twice daily for 5 days, 90% of lesions regressed in 6 weeks, and the remaining ones were smaller and less visible.
Case 5: Male, 56 years old with hand and neck verruca planae for 10 years and was treated with CO₂ laser but relapsed after the treatment. Those were brown papules of verruca planae scattered around hands, face and neck. After given methyltestosterone 1 tablet twice daily for 10 days, lesions on the face regressed in 2 months. A few less visible ones remained on the hand except one which was more visible but with reduced hyperplasia than before.
Case 6: Female, 17 years old with facial verruca planae for 3 months. They were brown papules of verruca planae on his face with scattered acnes. After given methyltestosterone 1 tablet once daily for 5 days, Vitamin B2 10mg three times daily for 15 days, Vitamin B6 10mg three times daily for 15 days and brimstone lotion used topically twice daily, her verruca planae was cured in 4 weeks with only acnes left on her face.
Case 7: Female, 21 years old with facial verruca planae. She was neither responsive to the treatment of anti-viral medications nor to a topical use of herbal medications. After given methyltestosterone 1 tablet twice daily for 5 days, she was cured upon follow-up visit 8 weeks later.
Case 8: Female, 18 years old with facial verruca planae for one year. After Given methyltestosterone 1 tablet once daily for 5 days, she was cured upon follow-up visit 8 weeks later.
Case 9: Male, 43 years old with facial brown papules, diagnosed as verruca planae 5 years ago but relapsed after a laser treatment. After Given methyltestosterone 1 tablet three times daily for 5 days, his lesions regressed mostly with only a few less visible, light brownish ones remaining.
Case 10: Male 37 years old with flat papules on the right forehead and blepharons, misdiagnosed as neurodermatitis. After given methyltestosterone 1 tablet three times daily for 4 days, the number of papules had reduced in 2 weeks and regressed completed 3 weeks after the treatment.
Case 11: Male, 29 years old with painless and itchless vegetations on penis for two weeks. There were 2 red lesions around the coronary sulcus and 5 positive spots for acetowhitening test. After a laser treatment followed by administering methyltestosterone 1 tablet three times daily for 5 days and 1 tablet twice daily for additional 5 days, the lesions did not relapse in 6 months after the treatment.
Case 12: Male, 36 years old with pain and itch around anus. There were three vegetations, one with 4-5mm in diameter and two with 1-2mm in diameter, all positive for the acetowhitening test. After a CO₂ laser treatment followed by administering methyltestosterone 1 tablet three times daily for 5 days and 1 tablet twice daily for additional 10 days, the lesions did not relapse in 6 months after the treatment.
Case 13: Female, 20 years old with a dozen vegetations around the vulva and anus, positive for the acetowhitening test. After a CO₂ laser treatment followed by administering methyltestosterone 1 tablet three times daily for 5 days and 1 tablet twice daily for additional 5 days, a few lesions did not heal in 4 weeks but no new vegetations appearing. The patient was then given 5‰ povidone iodine wash and wet compress. The wounds healed in 7 weeks and the patient was asked for a follow-up visit 6 months after the treatment.
Case 14: Male, 42 years old with 2 condylomas around anus. He was treated with a CO₂ laser method followed by methyltestosterone 1 tablet twice daily day for 10 days. No relapse occurred in a year.
Case 15: Male, 31 years old with 3 papules 4-5mm in diameter on prepuce entoplastron, positive for the acetowhitening test, and thus diagnosed as condyloma acuminatum. He was treated with a CO₂ laser followed by 1 tablet three times daily for 10 days. Mild edema and superficial erosion occurred one week after the treatment, and he was then given 5‰ povidone iodine wash and 5% amikacin sulfate to puff the wound. The wound was healed in 4 weeks with no scars left nor new vegetations growing.
Case 16: Male, 49 years old with condyloma acuminatum on coronary sulcus. He was treated with a freezing method followed by administering interferon three times daily both locally and systematically for 2 weeks. Yet several small granular new vegetations appeared around the original lessions and a few new ones on prepuce entoplastron after 1 month of the treatment. All new lesions were positive for the acetowhitening test. He was then treated with a CO₂ laser again and given methyltestosterone 1 tablet three times daily for 5 days and 1 tablet twice daily for additional 10 days. The wound was healed with a negative acetowhitening test in 6 weeks after the methyltestosterone treatment and no relapse occurred in one year.

These results demonstrate that treating verruca planae and condyloma acuminatum infected by HPV with methyltestosterone are far more effective than using other conventional methods. The invented treatment is especially efficacious to treat facial verruca planae and condyloma acuminatum following a laser or freezing treatment and effective to prevent the relapses as well. In addition, the treatment is convenient, painless, and inexpensive. The cost is only 1% to 1‰ of that using cytokines or immuno-modulators. Among the 60 condyloma acuminatum patients treated by operations followed by methyltestosterone, no relapses occurred in 6 months to one year. The two cases treated with interleukin II after operations did relapse but again the methyltestosterone treatment healed the lesions. According to "The Criteria of Diagnosis and Treatment for Condyloma Acuminatum" and "The Treatment Protocol of Condyloma Acuminatum" issued by the Bureau of Diseases Control of Chinese Ministry of Health and the Criteria of Treatment for Condyloma Acuminatum set by the WHO, all cases are considered as cure.

Because methyltestosterone has been used for several decades, the recommended dosage used in the invention is safe and non-toxic to humans.

In Summary, the present invention recommends that methyltestosterone be used to manufacture a pharmaceutical to treat and control diseases infected by HPV, especially verruca planae and condyloma acuminatum. The invention also provides a new insight in studying and preventing HPV-related diseases.

## Claims

1. Use of methyltestosterone for the manufacture of a pharmaceutical for treatment of HPV infections.

2. The use of claim 1, wherein said HPV infection is verruca planae.

3. The use of claim 1, wherein said HPV infection is condyloma acuminatum.

4. The use of anyone of claims 1-3, wherein said pharmaceutical is manufactured such as to be administered orally or hypoglossally.

5. The use of anyone of claims 1-3, wherein said methyltestosterone is comprised in a tablet.

6. The use of claim 5, wherein the content of methyltestosterone in said tablet is 5 mg/tablet.

7. The use of claim 6, wherein said tablet is designed to be administered hypoglossally, 1 tablet/time, two or three times/day, for 3-5 days consecutively.

8. The use of claim 6, wherein said tablet is designed to be administered orally, 1 tablet/time, three times/day, and 5 days consecutively, followed by 1 tablet/time, two times/day, for additional 5-10 days consecutively.

## Patentansprüche

1. Verwendung von Methyltestosteron für die Herstellung eines Medikamentes zur Behandlung von HPV-Infektionen.

2. Verwendung nach Anspruch 1, wobei die genannte HPV-Infektion Verruca Planae ist.

3. Verwendung nach Anspruch 1, wobei die genannte HPV-Infektion Condyloma Acuminatum ist.

4. Verwendung nach einem der Ansprüche 1-3, wobei das genannte Medikament so hergestellt ist, dass es oral oder hypoglossal verabreicht werden kann.

5. Verwendung nach einem der Ansprüche 1-3, wobei das genannte Methyltestosteron in einer Tablette enthalten ist.

6. Verwendung nach Anspruch 5, wobei der Gehalt an Methyltestosteron in der genannten Tablette 5 mg/Tablette beträgt.

7. Verwendung nach Anspruch 6, wobei die genannte Tablette dafür ausgelegt ist, hypoglossal verabreicht zu werden, 1 Tablette/Mal, zwei- oder dreimal täglich, während 3-5 aufeinanderfolgenden Tagen.

8. Verwendung nach Anspruch 6, wobei die genannte Tablette dafür ausgelegt ist, oral verabreicht zu werden, 1 Tablette/Mal, dreimal täglich und an 5 aufeinanderfolgenden Tagen, gefolgt von 1 Tablette/Mal, zweimal täglich, während weiteren 5-10 aufeinanderfolgenden Tagen.

## Revendications

1. Utilisation de méthyltestostérone pour la fabrication d'un composé pharmaceutique destiné au traitement d'infections à VPH.

2. Utilisation selon la revendication 1, dans laquelle ladite infection à VPH est une verrue plane.

3. Utilisation selon la revendication 1, dans laquelle ladite infection à VPH est un condylome acuminé.

4. Utilisation selon l'une quelconque des revendications 1 à 3, dans laquelle ledit composé pharmaceutique est fabriqué de sorte à être administré oralement ou par voie sublinguale.

5. Utilisation selon l'une quelconque des revendications 1 à 3, dans laquelle ladite méthyltestostérone est comprise dans un comprimé.

6. Utilisation selon la revendication 5, dans laquelle la teneur en méthyltestostérone dans ledit comprimé est de 5 mg/comprimé.

7. Utilisation selon la revendication 6, dans laquelle ledit comprimé est conçu pour être administré par voie sublinguale, 1 comprimé à la fois, deux ou trois fois par jour, pendant 3 à 5 jours consécutifs.

8. Utilisation selon la revendication 6, dans laquelle ledit comprimé est conçu pour être administré oralement, 1 comprimé à la fois, trois fois par jour et 5 jours consécutifs, suivi de 1 comprimé à la fois, deux fois par jour pendant 5 à 10 jours supplémentaires de manière consécutive.
